(19) Europäisches Patentamt
European Patent Office
Office européen des brevets

(11) Veröffentlichungsnummer: **0 372 332 B1**

(12) **EUROPÄISCHE PATENTSCHRIFT**

(45) Veröffentlichungstag der Patentschrift: **01.02.95**

(51) Int. Cl.[6]: **C12N 9/04**, C12P 7/26, //(C12N9/04,C12R1:225,1:72, 1:78,1:88)

(21) Anmeldenummer: **89121786.1**

(22) Anmeldetag: **25.11.89**

Die Akte enthält technische Angaben, die nach dem Eingang der Anmeldung eingereicht wurden und die nicht in dieser Patentschrift enthalten sind.

(54) **Mikrobiologisch hergestellte Diacetyl-Reduktase, Verfahren zu ihrer Gewinnung und ihre Verwendung.**

(30) Priorität: **03.12.88 DE 3840751**

(43) Veröffentlichungstag der Anmeldung: **13.06.90 Patentblatt 90/24**

(45) Bekanntmachung des Hinweises auf die Patenterteilung: **01.02.95 Patentblatt 95/05**

(84) Benannte Vertragsstaaten: **CH DE FR GB LI NL**

(56) Entgegenhaltungen:

**CHEMICAL ABSTRACTS, Band 73, 1970, Seite 33, Zusammenfassung Nr. 116599p, Columbus, Ohio, US; A.L. BRANEN et al.: "Diacetyl reductase of Lactobacillus casei"**

**7112699 EMBASE 88108294; I. VIDAL et al.: "Purification and classification of diacetyl-reducing enzymes from Staphylococcus aureus"**

**AGRIC. BIOL. CHEM., Band 51, Nr. 5, 1987, Seiten 1447-1448, Tokyo, JP; S. UI et al.: "Purification and properties of NADPH-linked diacetyl reductase (S-acetoin forming) from Bacillus polymyxa"**

(73) Patentinhaber: **FORSCHUNGSZENTRUM JÜLICH GMBH**
**Wilhelm-Johnen-Strasse**
**D-52425 Jülich (DE)**

(72) Erfinder: **Hummel, Werner, Dr.**
**Claudiustrasse 11**
**D-5177 Titz (DE)**
Erfinder: **Kula, Maria-Regina, Prof.Dr.**
**Selgenbusch 12**
**D-5162 Niederzier-Hambach (DE)**
Erfinder: **Boermann, Frank**
**Kaldenhausener Strasse 41**
**D-4150 Krefeld (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 372 332 B1

## Beschreibung

Gegenstand der Erfindung ist ein für die Bildung von Acetoin, insbesondere von (+)-Acetoin, brauchbares Enzym.

Acetoin ist Bestandteil der Aromakomponente, die z.B. von Streptococcus lactis ssp. diacetylactis gebildet wird. Im wesentlichen produziert dieses Bakterium neben Acetoin noch Diacetyl, 2,3-Butylen glycol und $C_2$-Verbindungen wie Ethanol, Essigsäure und Acetaldehyd (s. beispielsweise P. Schmitt et al., Appl. Microbiol. Biotechnol. (1988), 29: 430-436). Wegen dieser Aromastoffbildung wird das Bakterium häufig Milchprodukten (z.B. Butter) zugesetzt. Die Auftrennung dieser Komponenten z.B. zur Isolierung von Acetoin ist nicht üblich. Optisch aktive Acetoine, aber auch beispielsweise 2-Hydroxy-pentan-4-on sind zudem als chirale Bausteine für stereospezifische Synthesen interessant.

Chemisch hergestelles Acetoin ist ein racemisches Gemisch aus (+)- und (-)-Komponente, das in dieser Form beispielsweise als Lebensmittelzusatz nicht geeignet ist. Die Trennung des chemisch hergestellten Gemisches ist, wie bei den meisten Racematen, aufwendig und kostenintensiv.

In der Regel benutzt man hierfür Enzyme, die das eine Isomere umsetzen, mit nachfolgender Trennung der Verbindungen. Für Acetoin ist ein solches Verfahren nicht bekannt, überhaupt ist optisch aktives Acetoin kommerziell nicht erhältlich.

Ziel der Erfindung ist daher ein Verfahren zur enzymatischen Herstellung von Acetoin, insbesondere von (+)-Acetoin sowie ein dafür geeignetes Enzym und dessen Gewinnung.

Das erfindungsgemäße Verfahren zur Gewinnung von zusammen mit dem Coenzym NADH zur selektiven Reduktion von Diacetyl zu (+)-Acetoin befähigter Diacetyl-Reduktase ist dadurch gekennzeichnet, daß man einen Diacetyl-Reduktase produzierenden Stamm von Lactobacillus kefir, L. plantarum, L. brevis, L. buchneri oder Leuconostoc cremoris anzüchtet, die Zellmasse separiert und aufschließt und den erhaltenen Rohextrakt ggf. einer anschließenden Reinigung unterwirft.

Vorzugsweise wird zur Reinigung eine Hitzefällung bei 50 °C, eine Ionenaustauscher-Chromatographie, eine Gelfiltration sowie eine abschließende Feinreinigung mittels FPLC durchgeführt.

Die enzymatische Umsetzung von Diacetyl erfolgt nach der Gleichung

$$CH-\underset{\underset{O}{\|}}{C}-\underset{\underset{O}{\|}}{C}-CH_3 \quad + \quad NADH \quad + H^+ \longrightarrow CH_3-\underset{\underset{OH}{|}}{CH}-\underset{\underset{O}{\|}}{C}-CH_3 \quad + \quad NAD^+$$

in Gegenwart des Coenzyms NADH, das als Wasserstofflieferant für die Reduktion dient. Das Gleichgewicht der Reaktion begünstigt die Bildung von Acetoin, so daß - beispielsweise bei kontinuierlicher Reaktionsführung unter Regenerierung des Coenzyms - das Enzym für eine Acetoin-Synthese geeignet ist. Mit geringerer Aktivität kann auch (neben weiteren Verbindungen) das strukturell ähnliche Acetylaceton (2,4-Pentandion) als Substrat eingesetzt werden unter Bildung von 2-Hydroxy-4-pentanon.

Als Ausgangsmaterial für die Gewinnung der Diacetyl-Reduktase haben sich Stämme der in Tabelle 1 aufgeführten Lactobacillaceae als geeignet erwiesen.

In der Tabelle sind die Aktivitäten der Rohextrakte (feuchter Überstand nach Naßvermahlung und Zentrifugation) aufgeführt.

Als wesentliche Parameter der zur (+)-Acetoinbildung befähigten Diacetyl-Reduktase (nach internationaler Nomenklatur zu bezeichnen als Acetoin-Dehydrogenase; EC 1.1.1.X) sind zu nennen:

- Reaktivität in Gegenwart von NADH (Nicotinamidadenindinucleotid, reduziert) mit Diacetyl unter Bildung von (+)-Acetoin;
- Substratspezifität für Diacetyl, Pyruvat und Pyruvatethylester, daneben auch für andere Diketone wie Acetylaceton, Phenylpyruvat, Diacetylbenzol oder Hexandion;
- der $K_M$-Wert für die Reduktion von Diacetyl bei pH 4,25 beträgt 310 mM ($E_1$) bzw. 67 mM für $E_2$; für Acetylaceton beträgt der $K_M$-Wert 50 mM;
- spezifische Aktivität (hochgereinigt) von 1061 U/mg Protein;
- Molekulargewicht 66.000 ± 5.000 für die Diacetyl-Reduktase $E_1$ und 74.000 ± 5.000 für $E_2$;
- pH-Optimum für die Reduktionsreaktion 5 ± 1;
- Temperatur-Optimum: 70 °C;
- einwöchige Lagerfähigkeit bei 6 °C und einem pH-Wert zwischen 5 und 10 mit einer Restaktivität von 60%, speziell bei pH 9 einer Restaktivität von 100 %;
- Restaktivität nach 60 min bei 56 °C (pH 9) 90 %;
- starke Inhibierung durch $CuCl_2$, $FeCl_2$, $MgCl_2$, $HgCl_2$, Phenylhydrazin, 1,10-Phenanthrolin, 2,2-Dinitro-5,5-dithiobenzoesäure in 1 mM Konzentration, schwächere Inhibition durch PMSF (auf 30 % Restaktivität);

- unter Bedingungen, unter denen Enzyme in Untereinheiten aufspalten (Behandlung mit Natriumdodecylsulfat (SDS)), zeigt die Diacetyl-Reduktase keine Aufspaltung in Untereinheiten.

Tabelle 1: Aktivität der NADH-abhängige Reduktion von Acetoin und Acetylaceton

| Stamm (DSM Nr.) | | Akt.mit Diacetyl U/l | U/mg | mit Acetylaceton U/l | U/mg |
|---|---|---|---|---|---|
| Lactobacillen: | | | | | |
| Lc.plantarum | (20174) | 1439 | 0.34 | 396 | 0.11 |
| Lc.brevi | (20054) | 567 | 2.04 | 170 | 0.64 |
| Lc.buchneri | (20057) | 617 | 1.92 | | |
| Lc.kefir | (20587) | 2757 | 3.92 | 94 | 0.62 |
| Leuconos.cremoris | (20346) | 476 | 2.01 | | |

Zwar wird von A.L. Branen et al. (Canadian J. Microbiol. 16 (1970) 947-51) eine Diacetyl-Reductase aus Lactobacillus casei beschrieben, deren Aktivität zur Bildung optisch aktiver Formen von Acetoin jedoch unbekannt bleibt und deren spezifische Aktivität in gereinigter Form mit ~ 120 internationalen Einheiten/mg

erheblich hinter derjenigen der erfindungsgemäßen Diacetyl-Reductase zurückbleibt.

Besonders hohe spezifische Aktivitäten wurden im Rohextrakt von Lactobacillus kefir (DSM 20 587) gefunden.

Das nachfolgende Beispiel für die Enzymgewinnung bezieht sich daher auf dieses Bakterium. Seine Eigenschaften werden u.a. anhand der beigefügten Zeichnungen erläutert; es zeigen:

- Fig. 1: Wachstum und Enzymbildung von Lactobacillus kefir in Abhängigkeit von der Zeit; ●—● optische Dichte als Maß für das Wachstum, — Verlauf des pH-Wertes, ⊡ spezifische und ■ auf das Wachstum bezogene Enzymaktivität;
- Fig. 2: Abhängigkeit der Enzymaktivität vom pH-Wert in verschiedenen Puffersystemen; die jeweilige Aktivität wurde für das Enzym $E_1$ und $E_2$ bestimmt;
- Fig. 3: Abhängigkeit der Enzymaktivität von der Temperatur;
- Fig. 4: Stabilität der Diacetyl-Reduktase bei Lagerung in Puffern mit unterschiedlichen pH-Werten;
- Fig. 5: Gaschromatographische Auftrennung eines racemischen Acetoin-Gemisches (Fig. 5a) und des Produkts einer Umsetzung von Diacetyl mit der Diacetyl-Reduktase (Fig. 5b)

Beispiel 1: Gewinnung von Diacetyl-Reduktase

A. Züchtung von Lactobacillus kefir

Zur Enzymgewinnung wurde Lactobacillus kefir in folgendem Medium angezogen (pro 1 l):

| | |
|---|---|
| Glucose | 20 g |
| Hefeextrakt | 5 g |
| Universalpepton | 10 g |
| Fleischextrakt | 5 g |
| di-Ammoniumhydrogencitrat | 2 g |
| Natriumacetat | 5 g |
| Magnesiumsulfat | 0,1 g |
| Mangansulfat | 0,05 g |
| di-Kaliumhydrogenphosphat | 2 g |
| dest. $H_2O$ | 1 l |

Der pH-Wert dieser Lösung wurde auf 6,5 eingestellt, dann wurde für 15 min bei 121° C (2 bar) sterilisiert. Der Organismus wurde anaerob kultiviert, dazu reichte es aus, wenn das Medium mit $N_2$ überlagert wurde. Im 10 l-Maßstab wurde das Medium nach Erreichen der Bruttemperatur von 30° C mit 300 ml einer 24 Std alten Vorkultur beimpft. In einem solchen 10 l-Ansatz wurde beispielhaft der Verlauf der Enzymaktivität über der Zeit bestimmt, indem Proben zu verschiedenen Zeiten entnommen wurden, und die Aktivität der Diacetyl-Reduktase nach Aufschluß der Zellen bestimmt wurde. In Fig. 1 ist ein solcher Verlauf dargstellt, die Aktivität der Diacetyl-Reduktase erreicht nach kurzer Zeit einen maximalen Wert, der dann längere Zeit gehalten wird. Im 70 l-Maßstab wurde der Organismus bei Raumtemperatur kultiviert, nach 75 Stunden wurde bei einem pH-Wert von 4,15 und einer $OD_{660}$ von 4,12 durch Separieren 320 g feuchte Zellmasse geerntet. Die Zellmasse kann bei -20° C eingefroren gelagert werden, wobei über mehrere Monate ist kein Aktivitätsverlust erkennbar ist.

B. Enzymisolierung

Die Enzymfreisetzung aus den Zellen kann durch an sich bekannte Methoden (Ultraschall, Hochdruck-Homogenisation, Naßvermahlung u.a.) geschehen. Hier wurden die Zellen durch Naßvermahlung mit Glasperlen aufgeschlossen. Dazu wurde die Bakterienmasse (80 g) in 100 mM Tris-HCl-Puffer (pH 9,0) unter Zusatz von 0,1% 2-Mercaptoethanol suspendiert, so daß die Konzentration der Zellfeuchtmasse 40%ig war (Endvolumen 200 ml). Die Zellinhaltsstoffe wurden aus der gekühlten Suspension (4° C) durch einen mechanischen Aufschluß mit Hilfe einer Glasperlenmühle (Dyno-Mill, Fa. Bachofen) freigesetzt. Der 340 ml fassende Mahlbehälter wurde dazu mit Glasperlen (0,5 mm) gefüllt, so daß sich ein Schüttvolumen von 290 ml ergab (85%iger Füllgrad). Der Aufschluß wurde bei einer Rührwellendrehzahl von 2000 UpM durchgeführt. Der Kühlmantel und das Rührwellenlager wurden während des Laufes gekühlt.

80 g Bakterienfeuchtmasse ergaben 138 ml Rohextrakt mit einer Volumenaktivität von 136 U/ml und einem Proteingehalt von 29,4 mg/ml.

C. Enzymreinigung

- Hitzefällung

Die Acetoin-Dehydrogenaseaktivität des Lactobacillus erwies sich in Vorversuchen als ausgesprochen hitzestabil. Aus diesem Grunde bot sich eine selektive Hitzedenaturierung an, um Zellbruchstücke zu entfernen und eine erste Enzymanreicherung zu erreichen. Die aufgeschlossene Zellmasse wurde dazu 30-45 min bei 50 °C inkubiert, hierdurch wurden Aufreinigungsfaktoren von 1,8-2,1 mit Ausbeuten von 97-100% erzielt.

- Ionenaustauschchromatografie mit Q-Sepharose ff(R)

Die weitere Aufreinigung des Lactobacillus-Enzyms erfolgte durch Chromatographie an Q-Sepharose ff (R) (Ionenaustauscher; Fa. Pharmacia, Freiburg, BRD). Der Extrakt nach der Hitzedenaturierung mußte wegen unzureichender Bindung an das Austauscherharz jedoch vorher mit 50 mM Puffer entsalzt werden. Dies wurde mit einem ACA 54 Gel erreicht und ergab bei 98% Ausbeute einen zusätzlichen Aufreinigungsfaktor von 1,9. Am Kationenaustauscher wurde das Enzym durch Anlegen eines linearen Gradienten von 0-500 mM NaCl im 50 mM Tris-HCl-Puffer pH 9,0 bei 380 mM NaCl eluiert. Die vereinigten aktiven Fraktionen wiesen eine spezifische Aktivität von 78,5 U/mg Protein auf, was einem Aufreinigungsfaktor von 4 entspricht. Von der aufgetragenen Enzymmenge wurden 90% wiedergefunden.

- Gelfiltration mit ACA 54 (R)

Nach einer Volumenreduktion auf 4ml mittels einer Amicon 20000 Ultrafiltrationszelle wurde eine Gelfiltration an einem ACA-54-Gel (Fa. Pharmacia, Freiburg, BRD) durchgeführt. Hiermit konnte die spez. Aktivität um den Faktor 1,7 auf 130,5 U/mg gesteigert werden (Ausbeute von 97%).

D. Feinreinigung

- Fast Protein Liquid Chromatographie (FPLC) an Mono-Q (Ionenaustauscher)

Zur Feinreinigung des Enzyms wurde das aktive Eluat der Gelfiltration erneut einer Austauschchromatografie unterzogen, diesmal an einer Mono-Q Säule. 10 ml wurden in 1 ml Portionen auf die Säule aufgetragen und mit einem linearen NaCl-Gradienten von 150- 400 mM in 50 mM Tris-HCl-Puffer pH 9,0 eluiert. Bei diesem Verfahren konnten zwei aktive Enzyme voneinander getrennt werden, das eine (im folgenden E1 genannt) eluierte bei 230 mM NaCl, das andere (E2) bei 290 mM NaCl. Das Verhältnis von E1 zu E2 lag bei 4:1. Während E1 3-fach angereichert mit einer spez. Aktivität von 356 U/mg vorlag, wurde für E2 166 U/mg gemessen. Die Summe von E1 und E2 machte 86% der eingebrachten Enzymmenge aus.

- FPLC an Superose TM 12 (R)

Beide Enzyme wurden getrennt nach einer Aufkonzentrierung in einem Centricon 20000-Röhrchen (Ultrafiltration) über eine Superose TM12 (Fa. Pharmacia, Freiburg, BRD) weitergereinigt. E1 konnte so bis zu einer spez. Aktivität von 1061 U/mg, E2 bis 366 U/mg gereinigt werden. Dieser letzte Schritt verlief ohne nachweisbare Verluste.
Ein mit den am höchsten aufgereingten Fraktionen angefertigtes SDS-Gel ergab, daß E1 mit 1061 U/mg zu weit über 90 % aufgereinigt vorlag. Bei E2 hingegen wies das Gel noch mehrere Banden auf.

Die Reinigung der Diacetyl-Reduktaseaktivität ist in Tab. 2 zusammengefaßt.

Tabelle 2

| Reinigung der Lc.-Enzyme | | | | | | |
|---|---|---|---|---|---|---|
| Reinigungsschritt | | Vol. ml | Prot. mg | Total U | spez.Akt. U/mg | Ausb. % |
| Rohextrakt | | 10 | 294 | 1360 | 4,63 | 100 |
| Hitzedenaturierung | | 9,5 | 145 | 1346 | 9,18 | 99 |
| Entsalzung ACA 54 | | 28 | 76 | 1319 | 17,44 | 97 |
| Q-Sepharose | | 130 | 15 | 1184 | 78,5 | 87 |
| Ultrafiltration | | 4 | 15 | 1160 | 78,6 | 85 |
| Gelfil.ACA 54 | | 12 | 8,6 | 1125 | 130,5 | 83 |
| Mono-Q | E1<br>E2 | 6<br>5 | 2,2<br>1,2 | 774<br>194 | 356<br>166 | 71 |
| Centricon 20000 | | je0,5 | | | | |
| Superose | E1<br>E2 | 4,5<br>4,0 | 0,73<br>0,53 | 774<br>194 | 1061<br>366 | 71 |

E. Charakterisierung der Diacetyl-Reduktase

- pH-Abhängigkeit der Umsetzung

Die Abhängigkeit der Aktivität vom pH-Wert wurde bestimmt, indem 50 µl einer 1,1 M Diacetyl-Lösung in 1070 µl 1M Puffers des entsprechenden pH-Wertes 2h bei 25°C in der Küvette inkubiert wurde. Daraufhin wurde überprüft, ob nach diesem Zeitraum noch eine Diacetyl-spezifische Absorptionsänderung vorlag, welche nötigenfalls verrechnet wurde. Durch Zugabe von NADH-Lösung wurde die Coenzym-spezifische Fremdaktivität bestimmt. Nach Zugabe von Enzymlösung konnte die jeweilige Reaktionsgeschwindigkeit bestimmt werden.
Bei pH-Werten größer als 9,0 war keine Aktivität mehr meßbar, da das Diacetyl hier instabil ist.

Fig. 2 zeigt die pH-Abhängigkeit der Reduktion von Diacetyl durch die Lactobacillus kefir-Enzyme E1 und E2. Aus der Fig. 2 geht hervor, daß nicht nur der pH-Wert entscheidend für die Reaktiongeschwindigkeit, sondern auch die Art des Puffers. So weist E2 zum Beispiel im Tris-HCl-Puffer pH 7,5 gegenüber KPi-Puffer des gleichen pH-Wertes die 1,8-fache Aktivität auf.

Die Enzyme E1 und E2 zeigen eine ähnliche pH-Charakteristik, was wohl auf die pH-abhängige Konformationsänderung des Diacetyls zurückzuführen ist. Unterschiede liegen im Maximum: während E1 im Acetat-Puffer pH 4,25 die größte Aktivität aufweist, liegt das Maximum von E2 bei pH 5,0 im KPi-Puffer. (KPi-Puffer = Kaliumphosphat-Puffer).

Die Oxidationsreaktion von Acetoin mit NAD als Coenzym war trotz Variation des pH-Wertes und der Testkonzentrationen von Enzym, Coenzym und Substrat in keinster Weise meßbar.

- Temperaturoptimum der Enzymaktivität

Zur Bestimmung des Temperaturverhaltens wurden die Testansätze in einem temperierbaren Küvettenhalter bis zum Erreichung der gewünschten Temperatur inkubiert. Die Reaktion wurde durch Zugabe von Enzymlösung gestartet. Die Lactobacillus kefir -Aktivität wurde nach 20-facher Anreicherung eingesetzt, bei der E1 und E2 noch nicht voneinander getrennt waren. Die Fig. 3 zeigt die Abhängigkeit der Enzymaktivität von der jeweiligen Reaktionstemperatur.
Die Aktivität der Diacetyl-Reduktase nimmt bis zu einer Temperatur von 70°C zu.

- Einfluß verschiedener Metallkationen und Inhibitoren

Um das Verhalten der Diacetyl-reduzierenden Aktivität in Gegenwart von Metallkationen und Hemmstoffen zu bestimmen, wurden die entsprechenden Substanzen in einer Konzentration von 1 mM dem Assay

zugefügt und nach einer Inkubationszeit von 5 min bei 20 °C die Aktivitäten bestimmt. Das Absorptionsverhalten des Diacetyls machte es notwendig, zwei Blindwerte zu bestimmen, die zum einen unter Austausch von Enzymlösung, zum anderen durch Austausch von Substratlösung gegen Puffer ermittelt wurden. Tab. 3 faßt die Ergebnisse zusammen.

Tabelle 3

| Einfluß verschiedener Metallkationen und Inhibitoren | |
|---|---|
| Metallkationen / Hemmstoffe | Restaktivität des Lc.Enzyms / % |
| $BaCL_2$ | 102 |
| $CaCl_2$ | 96 |
| $CoCl_2$ | 102 |
| $CuCl_2$ | 0 |
| $FeCl_3$ | 82 |
| $FeCl_2$ | 0 |
| $MgCl_2$ | 0 |
| $MnCl_2$ | 91 |
| $NiCl_2$ | 100 |
| $HgCl_2$ | 0 |
| $ZnCl_2$ | 85 |
| L-Cycloserin | 100 |
| Phenylhydrazin | 0 |
| Jodacetamid | 99 |
| Jodessigsäure | 102 |
| 1.10.Phenantrolin | 0 |
| Diethylmalonsäure | 100 |
| 2.2 Bipyridin | 19 |
| 2.2 dinitro 5.5-dithiobenzoesäure | 0 |
| Gluthathion | 103 |
| Dithiothreitol | 106 |
| 1.4 dithioerythrit | 72 |
| p-Hydroximercuribenzoat | 79 |
| N-Ethylmaleinimid | 112 |
| EDTA | 94 |
| Triton X-100 | 113 |
| PMSF | 29 |

- Einfluß von Mangan-Ionen auf die Enzymstabilität

Das Enzym wurde für 1 h im Eisbad mit verschiedenen Manganchloridkonzentrationen inkubiert. Daraufhin wurde die jeweilige Aktivität bestimmt und nach 15 und 30 min durch erneute NADH Zugabe abermals die Aktivitäten bestimmt. Tabelle 4 zeigt, daß ein Zusatz von 5 mM Manganchlorid das Enzym deutlich stabilisiert.

Tabelle 4

| Abhängigkeit der Langzeitaktivität von der Mangankonzentration | | | |
|---|---|---|---|
| Mangankonzentration | rel.Aktivität / % | | |
| | t=0 | 15min | 30min |
| Vergleich ohne Mn | 100 | 50 | 0,3 |
| 1 mM | 100 | 69 | 0,3 |
| 2 mM | 100 | 79 | 25 |
| 5 mM | 100 | 88 | 39 |
| 10 mM | 100 | 46 | 19 |

- Einfluß des pH-Wertes auf die Lagerstabilität des Enzyms

Die pH-Stabilität der aufgereinigten Lc.-Enzyme wurde getestet, indem die Enzymlösungen 1:10 in sterilisiertem 1M Puffer des entsprechenden pH-Wertes verdünnt und für eine Woche bei 6°C inkubiert wurden. Fig. 4 zeigt den Einfluß der Lagerung bei verschiedenen pH-Werten auf die Enzymaktivität. Beide Enzyme zeigen breite Stabilitätsmaxima in einem pH-Bereich von 5 bis 9 bzw. 10,5 bei E1, mit höchsten Werten bei pH 9,0 für E1 (100%) und pH 7,0 für E2 (98%). Die verzeichneten Verluste waren dennoch höher als erwartet. Dies ist vermutlich darauf zurückzuführen, daß die ohnehin recht 'dünnen' Enzymlösungen mit weniger als 0,5 mg/ml Protein noch einmal 1:10 verdünnt wurden. Andere Enzympräperate konnten unter Zusatz von 43% Glycerin verlustfrei über mehrere Wochen bei 6°C und auch bei -18°C gelagert werden.

- Bestimmung des Molekulargewichtes für die Diacetyl-Reduktase $E_1$ und $E_2$

Die Molekulargewichte der Enzyme wurden gelchromatografisch an Superose TM 12 bestimmt. Mit Proteinstandards im Molmassenbereich von 12300 (Cytochrom C) bis 450000 (Ferritin) wurde die Säule für 50 mM KPi-Puffer unter Zugabe von 150 mM NaCl bei pH 7,5 kalibriert. Mit Hilfe einer Eichgerade konnten für E1 eine Molmasse von 66000 und für E2 eine von 74000 bestimmt werden. Bei einer SDS-Elektrophorese, also unter Bedingungen, unter denen Enzyme in Untereinheiten zerfallen, wurde für beide Enzyme eine Molmasse von 77000 bestimmt. Die Streuung der Eichproteine hat dabei gezeigt, daß die aufgetretenen Abweichungen innerhalb der Genauigkeitsgrenzen der Methode liegen. Sicher zu sagen ist jedoch, daß beide Enzyme monomer vorliegen.

- Abhängigkeit derEnzymaktivität von der Substratkonzentration (Bestimmung des $K_M$-Wertes)

Zur Bestimmung des $K_M$-Wertes wurde die Aktivität der beiden Lactobacillus-Enzyme in Abhängigkeit von der Substratkonzentration gemessen. Bei E1 und E2 tritt Substratsättigung erst oberhalb von 1M auf, wobei zu bemerken ist, daß bei so hohen Diacetyl-Konzentrationen eine Denaturierung der Enzyme unter Aktivitätsverlust auftreten kann.
Die lineare Regression der Lineweaver-Burk-Kurve ergab folgende $K_m$-Werte:

E1 : $K_m$ = 310
E2 : $K_m$ = 67 mM

- Substratspektrum der Diacetyl-Reduktase $E_1$ und $E_2$

Mit den am höchsten aufgereinigten Präparaten der Lc.-Enzyme E1 und E2 wurden verschiedene Substrate getestet. Durch Variation der Konzentration konnte für jedes Substrat die maximale Umsatzgeschwindigkeit und der $K_M$-Wert ermittelt werden. Tab. 5 faßt die Ergebnisse zusammen.

Tabelle 5

| Substratspektrum | | | | |
|---|---|---|---|---|
| Substrat | rel.Vmax | | $K_m$ / M | |
| | EI | E2 | EI | E2 |
| Diacetyl | 100 | 100 | 3,1 x $10^{-1}$ | 6,7 x $10^{-2}$ |
| NADH | 100 | 100 | 3,0 x $10^{-5}$ | 3.6 x $10^{-5}$ |
| Acetylaceton | 8 | - | 5,0 x $10^{-2}$ | - |
| 2,5-Hexandion | 7 | - | 2,6 x $10^{-2}$ | - |
| Pyruvat | 104 | 14 | 5,7 x $10^{-5}$ | 1,3 x $10^{-5}$ |
| Pyruvatethylester | 109 | 77 | 2,4 x $10^{-3}$ | 2,1 x $10^{-3}$ |
| $\beta$-Phenylpyruvat | 17 | 22 | 4,8 x $10^{-4}$ | 1,3 x $10^{-4}$ |
| 3-Ketobuttersäureethylester | - | - | - | - |
| 5-Cl-2-Pentanon | - | - | - | - |
| 1,3-Cyclohexandion | - | - | - | - |
| Diacetylbenzol | 1,3 | | - | - |
| $\alpha$-Ketoglutarsäure | - | - | - | - |

Anhand der vorstehenden Daten wäre zu vermuten, daß es sich bei der Diacetyl-Reduktase um eine häufig vorkommende Lactat-Dehydrogenase handelt. Verwendet man jedoch käufliche Lactat-Dehydrogenasen, so stellt man fest, daß mit diesen Diacetyl nicht umgesetzt wird.

- Nachweis der Stereospezifität durch Oxidation von D(-)- und L(+)-Milchsäure

Die Möglichkeit, die Stereospezifität einer Dehydrogenase (oder Reduktase) durch Oxidation der jeweiligen Hydroxyverbindung zu bestimmen, ist für die Diacetyl-Reduktion nicht durchführbar; es fehlen die optisch reinen (+)- und (-)-Acetoine und es hat sich bei diesem Enzym gezeigt, daß die Rückreaktion nicht stattfindet. Da das Enzym - wie in Tab. 5 gezeigt - aber auch Pyruvat reduzieren kann, hat man hier die Möglichkeit, die Stereospezifität anhand des Substratpaares (+)-und (-)-Lactat zu ermitteln. In Testansätzen mit D(-)- bzw. L(+)-Milchsäure und $NAD^+$ in Kaliumphosphatpuffer (pH 7,0) konnte D(-)-Milchsäure von beiden Enzymen $E_1$ und $E_2$ recht gut umgesetzt werden, L(-) -Milchsäure dagegen wurde von $E_1$ mit 5 %, und von $E_2$ mit 4 % dieser Aktivität umgesetzt.

<u>Beispiel 2</u>

Enzymkatalysierte Herstellung von Acetoin in batch-Ansätzen und Produktnachweis

Mehrere batch-Umsetzungen mit dem Enzym $E_1$ bzw. $E_2$ wurden unter Coenzymregenerierung durchgeführt. Zur Regenerierung wurden Formiat und die Formiat-Dehydrogenase eingesetzt. Eingesetzt wurden zum einen ein Enzympräparat, bei dem die beiden Enzyme $E_1$ und $E_2$ noch nicht voneinander getrennt waren, zum anderen hoch angereicherte Präparate von E1 und E2. Die Komponenten und ihre Konzentrationen sind folgender Tabelle 6 zu entnehmen.

Tabelle 6

| Umsetzungen von Diacetyl zu Acetoin in Gegenwart eines NADH-regenerierenden Enzymsystems | | | |
|---|---|---|---|
| | 1 Lc.-Enz. | 2 E1 | 3 E2 |
| Gesamtvolumen | 1100 l | 1000 l | 1000 l |
| pH-Wert | 5,4 | 7,5 | 7,5 |
| Enzymmenge | 6 U | 2 U | 2 U |
| [ Diacetyl ] | 200 mM | 100 mM | 100 mM |
| [ NADH ] | 5 mM | 5 mM | 5 mM |
| Enzymmenge FDH | 12 U | 2 U | 2 U |
| [ Formiat ] | 300 mM | 500 mM | 500 mM |
| Dauer | 16 h | 8 h | 8 h |
| Umsatz | 85 % | 52 % | 65 % |
| gem. Drehwinkel | | 0,074 ° | 0,085 ° |
| Messkonz. | | 12,1 mM | 15,0 mM |
| spez.Drehung | | 71 ° | 65 ° |
| Enatioselektiv. | 94 % | | |

Als analytisches Verfahren zur qualitativen und quantiativen Bestimmung des Produktes Acetoin in Gegenwart des Eduktes Diacetyl eignete sich sowohl die Dünnschichtchromatografie als auch die Gaschromatografie.

Dünnschicht-Chromatographie:

Damit konnte qualitativ die Bildung von Acetoin nachgewiesen werden. Aus Gründen der Detektion mußte für die dünnschichtchromatografische Auftrennung beider Stoffe eine Derivatisierung mit 2.4-Dinitrophenylhydra-zin zum gelb gefärbten Hydrazon durchgeführt werden. Auf einer Kieselgel-G Platte mit UV-Indikator unter Benutzung des Laufmittels Petrolether / Dioxan in einem Verhältnis 2:1 ergab sich für das Acetoinhydrazon ein $R_f$-Wert von 0,69 und für das Diacetylhydrazon ein $R_f$-Wert von 0,91.
Mit einer dünnschichtchromatografischen Auftrennung von Proben des ersten Ansatzes konnte das Produkt Acetoin nachgewiesen werden.

Gaschromatographie:

Die gaschromatografische Auftrennung ermöglicht eine Quantifizierung der Umsetzung, die Auftrennung erfolgte an einer gepackten Poropak-Q-Säule (Macherey und Nagel, Düren, BRD). Bei 180°isothermer Arbeitsweise, einer Injektor-/ Detektortemperatur von 200°C und einer Einspritzmenge von 1µl wässriger Lösung wurde Diacetyl nach 8,35 min und Acetoin nach 19,5 min eluiert. Die in Tab. 6 angegebenen Umsatz-Werte sind mit dieser Methode bestimmt worden.

Bestimmung der spezifischen Drehung:

Zur Bestimmung der spezifischen Drehung von Acetoin wurden von je 300 µ l der Batch-Ansätze E1 und E2 die Proteine mittels eines Centricon 10000-Röhrchens (Ultrafiltration) abgetrennt. Nach einer Verdünnung mit 1 ml $H_2O$ wurde in einem Polarimeter bei 578 nm in einer 1 dm-Küvette gemessen. Für die E1-Probe ergab sich ein Drehwinkel von +0,075° und für E2 einer von +0,085° (Blindwert ohne Protein: -0,003°). Mit den Umsatzraten von 52% und 65% konnte daraus eine spezifische Drehung von +71,0° (bei E1), bzw. +63,5° (bei E2) errechnet werden.
Vergleichend mit den Arbeiten von Discherl-Schöllig (1938), bei denen sich für das (-) Acetoin in wässriger Lösung -46 bis -48°,bei einigen Versuchen aber auch - 105° ergab, liegen die oben bestimmten Werte im zu erwartenen Bereich.

Bestimmung der Enantioselektivität

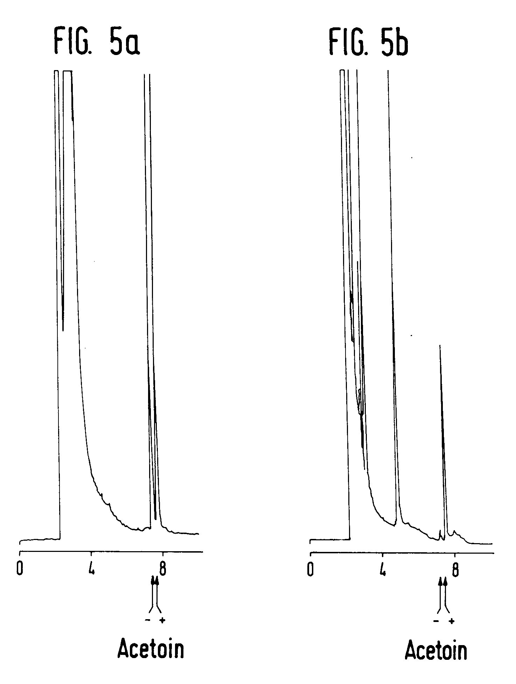

Die Enantioselektivität der Enzyme konnte mit einer Chiralsil-val Kapillarsäule (Macherey und Nagel, Düren, BRD) gaschromatographisch bestimmt werden. Dazu wurde das Produkt des Batch-Ansatzes 1 durch eine mehrstufige Extraktion aus der wässrigen Phase in eine organische Essigsäureethylester-Phase überführt. Nach einer Derivatisierung des Acetoins mit Isopropylisocyanat wies das entstandene Acetoinurethan genügend Affinität zur stationären L-Valin-tert.butylamid Phase der Säule auf, um in die optischen Enantiomere aufgetrennt zu werden. Bei einer isothermen Arbeitsweise von 120° wurde das linksdrehende Enantiomer nach 7,49 min eluiert, das rechtsdrehende nach 7,80 min. Bei 110° ergaben sich Retentionszeiten von 10,39 und 10,85 min. Die Abb. 7 zeigt die Auftrennung eines racemischen Gemisches von ± Acetoin (Fig. 5a) sowie die Auftrennung des Ansatzes 1 der Tab. 6 (Fig 5b). Das Chromatogramm macht deutlich, daß das aus Lactobacillus kefir isolierte Enzym zu 97 % das rechtsdrehende Acetoin-Enantiomer synthetisiert.

## Patentansprüche

**1.** Verfahren zur Gewinnung von zusammen mit dem Coenzym NADH zur selektiven Reduktion von Diacetyl zu (+)-Acetoin befähigter Diacetyl-Reduktase,
**dadurch gekennzeichnet,**
daß man einen Diacetyl-Reduktase produzierenden Stamm von Lactobacillus kefir, L. plantarum, L. brevis, L. buchneri oder Leuconostoc cremoris anzüchtet, die Zellmasse separiert und aufschließt und den erhaltenen Rohextrakt ggf. einer anschließenden Reinigung unterwirft.

**2.** Verfahren nach Anspruch 1,
**dadurch gekennzeichnet,**
daß man zur Reinigung eine Hitzefällung bei 50°C, eine Ionenaustauscher-Chromatographie, eine Gelfiltration sowie eine abschließende Feinreinigung mittels FPLC durchführt.

**3.** Zusammen mit dem Coenzym NADH zur selektiven Reduktion von Diacetyl zu (+)-Acetoin befähigte Diacetyl-Reduktase mit einer Aktivität von > 0,5 U/mg,
**gekennzeichnet durch**
folgende Parameter:
- Substratspezifität für Diacetyl, Pyruvat und Pyruvatethylester, daneben auch für andere Diketone wie Acetylaceton, Phenylpyruvat, Diacetylbenzol oder Hexandion;
- der $K_M$-Wert für die Reduktion von Diacetyl bei pH 4,25 beträgt 310 mM ($E_1$) bzw. 67 mM für $E_2$; für Acetylaceton beträgt der $K_M$-Wert 50 mM;
- spezifische Aktivität (hochgereinigt) von 1061 U/mg Protein;
- Molekulargewicht 66.000 ± 5.000 für Diacetyl-Reduktase $E_1$ und 74.000 ± 5.000 für $E_2$;
- pH-Optimum für die Reduktionsreaktion 5 ± 1;
- Temperatur-Optimum: 70°C;
- einwöchige Lagerfähigkeit bei 6° C und einem pH-Wert zwischen 5 und 10 mit einer Restaktivität von 60%, speziell bei pH 9 einer Restaktivität von 100%.
- Restaktivität nach 60 min bei 56°C (pH 9) 90%;
- starke Inhibierung durch $CuCl_2$, $MgCl_2$, $FeCl_2$, $HgCl_2$, Phenylhydrazin, 1,10-Phenanthrolin, 2,2-Dinitro- 5,5-dithiobenzoesäure in 1 mM Konzentration, schwächere Inhibition durch PMSF (auf 30% Restaktivität);
- unter Bedingungen, unter denen Enzyme in Untereinheiten aufspalten (Behandlung mit Natriumdodecylsulfat (SDS)), zeigt die Diacetyl-Reduktase keine Aufspaltung in Untereinheiten;
  erhältlich aus Lactobacillus kefir, Lactobacillus plantarum, Lactobacillus brevis, Lactobacillus buchneri oder Leuconostoc cremoris.

**4.** Verfahren zur Herstellung von (+)-Acetoin,
**dadurch gekennzeichnet,**
daß man Diacetyl enzymatisch mit einer nach Anspruch 1 oder 2 erhältlichen Diacetyl-Reduktase in Gegenwart von NADH umsetzt und das gebildete (+)-Acetoin isoliert.

**5.** Verfahren zur Herstellung von 2-Hydroxypentan-(4)-on
**dadurch gekennzeichnet,**

daß man eine Acetylaceton enthaltende wässrige Lösung enzymatisch mit einer nach Anspruch 1 oder 2 erhältlichen Diacetyl-Reduktase in Gegenwart von NADH umsetzt und das gebildete 2-Hydroxypentan-(4)-on isoliert.

## Claims

**1.** Process for the recovery of diacetyl reductase, together with the coenzyme NADH capable of selectively reducing diacetyl to (+)-acetoin,
**characterized in that**
a strain of Lactobacillus kefir, L. plantarum, L. brevis, L. buchneri or Leuconostoc cremoris producing diacetyl reductase is cultured, the cell mass is separated and digested, and the crude extract obtained is optionally subjected to a subsequent purification step.

**2.** Process according to claim 1,
**characterized in that**
purification is achieved by thermal precipitation at 50 °C, by ion exchange chromatography, gel filtration and final fine purification using FPLC.

**3.** Diacetyl reductase with an activity of > 0.5 U/mg, together with the coenzyme NADH capable of selectively reducing diacetyl to (+)-acetoin,
**characterized by**
the following parameters:
- substrate specificity for diacetyl, pyruvate and ethyl pyruvate, as well as for other diketones such as acetylacetone, phenylpyruvate, diacetylbenzene or hexane dione;
- the $K_M$ value for the reduction of diacetyl at pH 4.25 is 310 mM ($E_1$) and 67 mM for $E_2$; for acetylacetone the $K_M$ value is 50 mM;
- specific activity (highly purified) of 1061 U/mg of protein;
- molecular weight 66,000 ± 5,000 for diacetyl reductase $E_1$ and 74,000 ± 5,000 for $E_2$;
- pH optimum für the reduction reaction 5 ± 1;
- temperature optimum: 70 °C;
- one-week storability at 6 °C and a pH value between 5 and 10 with a residual activity of 60 %, specifically a residual activity of 100 % at pH 9;
- 90 % residual activity after 60 min at 56 °C (pH 9);
- strong inhibition by $CuCl_2$, $MgCl_2$, $FeCl_2$, $HgCl_2$, phenylhydrazine, 1,10-phenanthroline, 2,2-dinitro-5,5-dithiobenzoic acid in 1 mM concentration, weaker inhibition by PMSF (to 30 % residual activity);
- diacetyl reductase does not show any splitting into subunits under conditions under which enzymes split into subunits (treatment with sodium dodecylsulphate (SDS));
  obtainable from Lactobacillus kefir, Lactobacillus plantarum, Lactobacillus brevis, Lactobacillus buchneri or Leuconostoc cremoris.

**4.** Process for the production of (+)-acetoin,
**characterized in that**
diacetyl is enzymatically converted by a diacetyl reductase obtainable according to claim 1 or 2 in the presence of NADH and the (+)-acetoin formed is isolated.

**5.** Process for the production of 2-hydroxypentane-(4)-on,
**characterized in that**
an aqueous solution containing acetylacetone is enzymatically converted by a diacetyl reductase obtainable according to claim 1 or 2 in the presence of NADH and the 2-hydroxypentane-(4)-on formed is isolated.

## Revendications

**1.** Procédé pour l'obtention d'une diacétyle-réductase capable à la réduction sélective de diacétyle en (+)-acétoïne en présence de la coenzyme NADH,
**caractérisé par le fait**
qu'on cultive une souche de Lactobacillus kefir, L. plantarum, L. brevis, L. buchneri ou Leuconostoc

cremoris produisant une diacétyle-réductase, qu'on sépare et désintègre la masse cellulaire et, le cas échéant, soumet l'extrait cru obtenu à une purification ultérieure.

**2.** Procédé suivant la revendication 1,
**caractérisé par le fait**
que pour la purifaction on effectue une précipitation à chaud à 50 °C, une chromatographie échangeuse d'ions, une filtration sur gel ainsi qu'une purification poussée finale au moyen de FPLC.

**3.** Une diacétyle-réductase d'une activité de > 0,5 U/mg, capable à la réduction sélective de diacétyle en (+)-acétoïne en présence de la coenzyme NADH,
caractérisé par
les paramètres suivants:
- la spécifité vis-à-vis du substrat pour le diacétyle, le pyruvate et le pyruvate d'éthyle, ainsi que pour d'autres dicétones comme l'acétylacétone, le phénylpyruvate, le diacétylbenzène ou le dione d'hexane;
- la valeur $K_M$ pour la réduction de diacétyle à pH 4,25 est de 310 mM ($E_1$) et de 67 mM pour $E_2$; la valeur $K_M$ pour l'acétylacétone est de 50 mM;
- activité spécifique (fortement purifiée) de 1061 U/mg de proteïne;
- poids moléculaire 66.000 ± 5.000 pour la réductase diacétyle $E_1$ et 74.000 ± 5.000 pour $E_2$;
- l'optimum de pH pour la réaction de réduction: 5 ± 1;
- température optimale: 70 °C;
- supportant le stockage d'une semaine à 6 °C et à une valeur pH entre 5 et 10 avec une activité résiduelle de 60 %, notamment une activité résiduelle de 100 % à pH 9;
- activité résiduelle après 60 min à 56 °C (pH 9): 90 %;
- inhibition forte par $CuCl_2$, $MgCl_2$, $FeCl_2$, $HgCl_2$, la phénylhydrazine, 1,10-phénanthroline, l'acide 2,2-dinitro-5,5-dithiobenzoïque à une concentration de 1 mM, inhibition plus faible par PMSF (à une activité résiduelle de 30 %);
- dans des conditions de décomposition d'enzymes en unités secondaires (traitement au sulfate dodécylique de sodium (SDS)), la réductase diacétyle ne montre aucune décomposition en unités secondaires; elle peut être obtenue à partir de Lactobacillus kefir, Lactobacillus plantarum, Lactobacillus brevis, Lactobacillus buchneri ou Leuconostoc cremoris.

**4.** Procédé pour la production de (+)-acetoïne,
**caractérisé par le fait**
qu'on transforme le diacétyle de manière enzymatique à l'aide d'une diacétyle-réductase obtenue suivant l'une des revendications 1 ou 2 en présence de NADH et qu'on isole la (+)-acétoïne formée.

**5.** Procédé pour la production de 2-hydroxypentane-(4)-on,
**caractérisé par le fait**
qu'on transforme une solution aqueuse contenant de l'acétylacétone de manière enzymatique à l'aide d'une diacétyle-réductase obtenue suivant l'une des revendications 1 ou 2 en présence de NADH, et qu'on isole le 2-hydroxypentane-(4)-on formé.

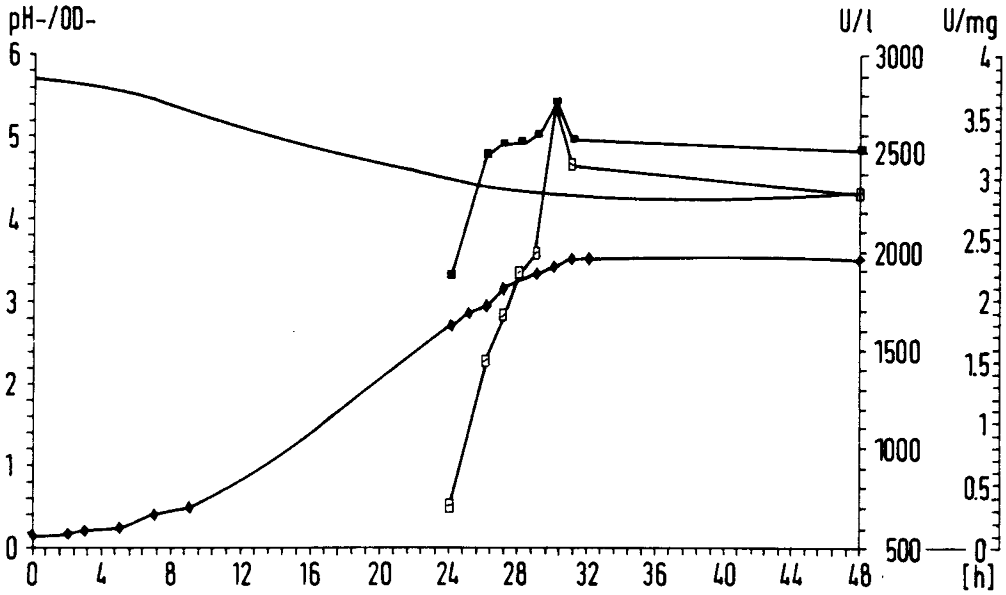
pH-/OD-
U/l
U/mg
6
5
4
3
2
1
0
3000
2500
2000
1500
1000
500
4
3.5
3
2.5
2
1.5
1
0.5
0
0
4
8
12
16
20
24
28
32
36
40
44
48
[h]

FIG. 1

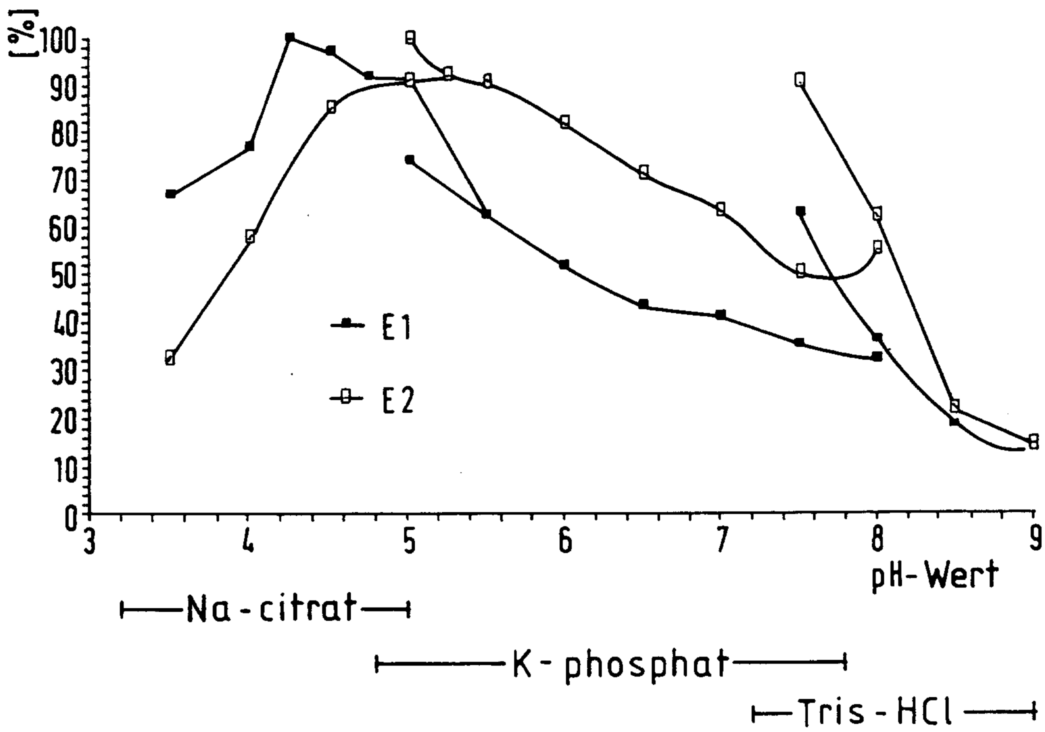
[%]
100
90
80
70
60
50
40
30
20
10
0
3
4
5
6
7
8
9
pH-Wert
E1
E2
Na-citrat
K-phosphat
Tris-HCl

Fig. 2

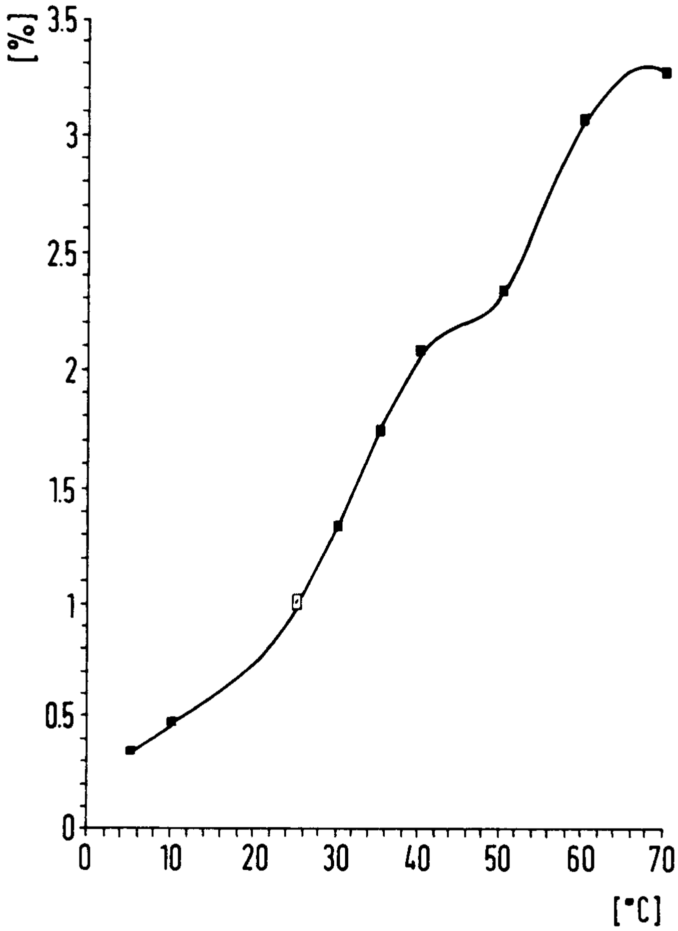


FIG. 3

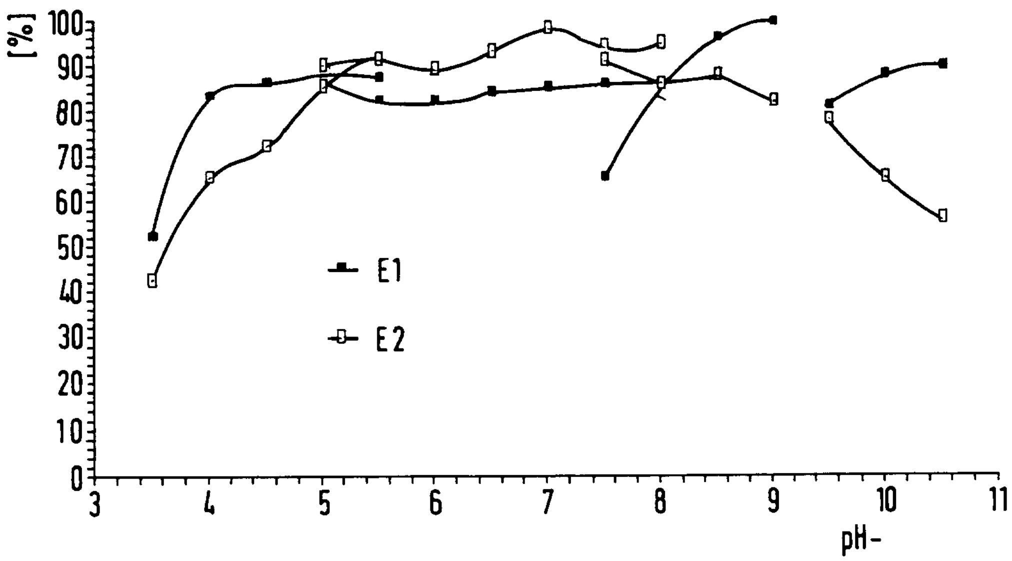


FIG. 4

FIG. 5a

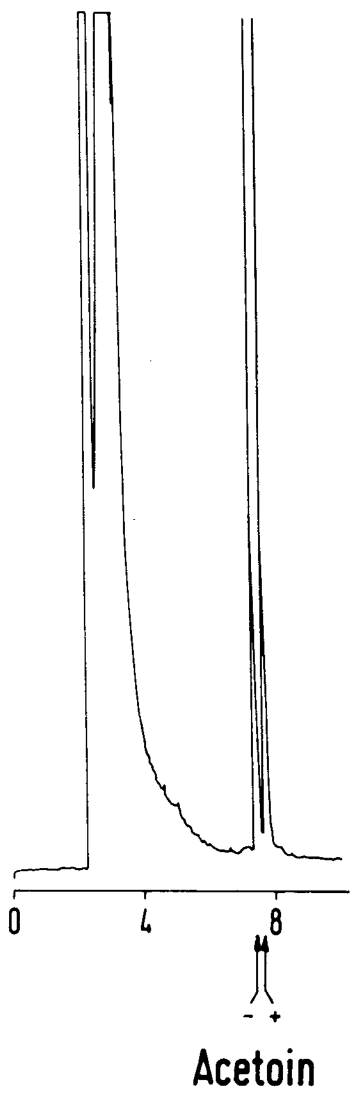


FIG. 5b